# EUROPÄISCHE PATENTSCHRIFT

(11) **EP 2 667 793 B1**
(45) Veröffentlichungstag und Bekanntmachung des Hinweises auf die Patenterteilung: **16.03.2016**
(21) Anmeldenummer: 11805534.2
(22) Anmeldetag: 27.12.2011
(51) Int. Cl.: A61B 17/14, B27B 19/00

(54) **TRAGBARE WERKZEUGMASCHINE MIT WERKZEUGSPANNVORRICHTUNG**
PORTABLE MACHINE TOOL WITH TOOL CHUCKING DEVICE
MACHINE-OUTIL PORTABLE AVEC DISPOSITIF DE SERRAGE D'OUTIL

(30) Priorität: 25.01.2011 DE 102011003103
(43) Veröffentlichungstag der Anmeldung: 04.12.2013
(73) Patentinhaber: Robert Bosch GmbH, 70442 Stuttgart (DE)
(72) Erfinder: FANKHAUSER, Marcel, CH-3011 Bern (CH); LUESCHER, Bruno, CH-4800 Zofingen (CH); GYORI, Szabolcs, 70771 Leinfelden (DE)
(86) Internationale Anmeldenummer: PCT/EP2011/074105
(87) Internationale Veröffentlichungsnummer: WO 2012/100893

(56) Entgegenhaltungen:
- EP-A1- 1 752 256
- EP-A2- 0 776 634
- WO-A1-95/13020
- CH-A5- 563 847
- GB-A- 2 285 011
- US-A- 1 705 205
- US-A1- 2005 192 585

## Beschreibung

### Stand der Technik

Es sind bereits Werkzeugspannvorrichtungen, insbesondere Oszillationswerkzeugspannvorrichtungen, bekannt, die eine Spanneinheit aufweisen. Die Spanneinheit weist hierbei ein stiftförmiges Spannelement zu einem Festspannen eines Bearbeitungswerkzeugs in einer Axialrichtung und einen an dem Spannelement angeordneten Spannkopf auf.

Offenbarung der Erfindung Die Erfindung geht aus von einer tragbaren Werkzeugmaschine gemäß dem Oberbegriff des Patentanspruchs 1 mit einer Werkzeugspannvorrichtung, insbesondere von einer Oszillationswerkzeugspannvorrichtung, mit zumindest einer Spanneinheit, die zumindest ein stiftförmiges Spannelement zu einem Festspannen eines Bearbeitungswerkzeugs in einer Axialrichtung sowie zumindest einen an dem Spannelement angeordneten Spannkopf aufweist.

Eine derartige Werkzeugmaschine geht beispielsweise aus der EP 0 776 634 A2 hervor. Die Erfindung sieht eine tragbare Werkzeugmaschine gemäß dem Patentanspruch 1 vor.

Die Werkzeugspannvorrichtung weist zumindest eine Bewegungswandlungseinheit auf, die dazu vorgesehen ist, zumindest den Spannkopf in Abhängigkeit einer Bewegung des Spannelements zumindest im Wesentlichen entlang der Axialrichtung in zumindest eine von der Axialrichtung abweichende Richtung zu bewegen. In diesem Zusammenhang soll der Begriff "vorgesehen" speziell ausgestattet und/oder speziell ausgelegt definieren. Unter einer "Spanneinheit" soll hier insbesondere eine Einheit verstanden werden, die ein Bearbeitungswerkzeug mittels eines Formschlusses und/oder mittels eines Kraftschlusses entlang der Axialrichtung sichert, insbesondere an einer Werkzeugaufnahme einer tragbaren Werkzeugmaschine. In einem Spannmodus der Spanneinheit wirkt eine Spannkraft entlang der Axialrichtung auf das Bearbeitungswerkzeug. Unter einem "stiftförmigen Spannelement" soll hier insbesondere ein Spannelement verstanden werden, das in einem montierten Zustand eine Längserstreckung entlang der Axialrichtung aufweist, die größer ist als eine Quererstreckung des Spannelements entlang einer senkrecht zur Axialrichtung verlaufenden Richtung. Insbesondere ist die Längserstreckung mehr als doppelt so groß als die Querstreckung des Spannelements, bevorzugt mehr als vier Mal so groß und besonders bevorzugt mehr als sechs Mal so groß. Bevorzugt ist das stiftförmige Spannelement zumindest teilweise als Hohlkörper ausgebildet. Besonders bevorzugt weist das Spannelement zumindest zwei als Schenkel ausgebildete Teilbereiche auf, die zumindest teilweise entlang einer zumindest im Wesentlichen senkrecht zur Axialrichtung verlaufenden Richtung relativ zueinander beabstandet angeordnet sind. Vorzugsweise ist das Spannelement verliersicher in einer Hohlwelle der tragbaren Werkzeugmaschine angeordnet. Der Begriff "Axialrichtung" soll hier insbesondere eine Richtung definieren, die bevorzugt zumindest im Wesentlichen parallel zu einer Schwenkachse und/oder Rotationsachse einer zum Antrieb des Bearbeitungswerkzeugs vorgesehenen Antriebswelle und/oder Spindel einer tragbaren Werkzeugmaschine verläuft. Unter "im Wesentlichen parallel" soll hier insbesondere eine Ausrichtung einer Richtung relativ zu einer Bezugsrichtung, insbesondere in einer Ebene, verstanden werden, wobei die Richtung gegenüber der Bezugsrichtung eine Abweichung insbesondere kleiner als 8°, vorteilhaft kleiner als 5° und besonders vorteilhaft kleiner als 2° aufweist.

Unter einem "Spannkopf" soll hier ein Element verstanden werden, das zumindes zwei relativ zueinander bewegliche Teilbereiche aufweist, die zum Festspannen des Bearbeitungswerkzeugs in Axialrichtung zumindest an einer Teilfläche des Bearbeitungswerkzeugs anliegen und das Bearbeitungswerkzeug mit einer Spannkraft entlang der Axialrichtung beaufschlagen. Der Begriff "Bewegungswandlungseinheit" soll hier insbesondere eine Einheit definieren, die einen Mechanismus umfasst, mittels dessen eine Bewegung eines ersten Elements, insbesondere eine translatorische Bewegung des ersten Elements, in eine Bewegung eines weiteren Elements, insbesondere in eine Schwenkbewegung und/oder eine translatorische Bewegung des weiteren Elements, umgewandelt werden kann. Bevorzugt wird mittels der Bewegungswandlungseinheit eine translatorische Bewegung des Spannelements in eine Schwenkbewegung und/oder in eine translatorische Bewegung des Spannkopfs umgewandelt. Besonders bevorzugt wird der Spannkopf mittels der Bewegungswandlungseinheit zumindest im Wesentlichen entlang einer Hauptbewegungskomponente bewegt, die zumindest im Wesentlichen senkrecht zur Axialrichtung verläuft. Der Ausdruck "im Wesentlichen senkrecht" soll hier insbesondere eine Ausrichtung einer Richtung relativ zu einer Bezugsrichtung definieren, wobei die Richtung und die Bezugsrichtung, insbesondere in einer Ebene betrachtet, einen Winkel von 90° einschließen und der Winkel eine maximale Abweichung von insbesondere kleiner als 8°, vorteilhaft kleiner als 5° und besonders vorteilhaft kleiner als 2° aufweist. Unter "im Wesentlichen entlang" soll hier insbesondere ein Verlauf einer Richtung entlang einer Bezugsrichtung verstanden werden, wobei die Richtung gegenüber der Bezugsrichtung eine Abweichung insbesondere kleiner als 8°, vorteilhaft kleiner als 5° und besonders vorteilhaft kleiner als 2° aufweist. Mittels der erfindungsgemäßen Ausgestaltung der Werkzeugspannvorrichtung kann vorteilhaft ein einfach realisierbarer Spannvorgang erreicht werden. Ferner kann konstruktiv einfach ein Festspannen des Bearbeitungswerkzeugs an einer Werkzeugaufnahme der tragbaren Werkzeugmaschine, insbesondere der tragbaren Werkzeugmaschine mit einer oszillierend antreibbaren Spindel, erreicht werden.

Ferner wird vorgeschlagen, dass die Bewegungswandlungseinheit zumindest ein Bewegungswandlungselement aufweist, das dazu vorgesehen ist, zumindest in einem Spannmodus der Spanneinheit den Spannkopf zur Erzielung einer axialen Überdeckung des Spannkopfs und des Bearbeitungswerkzeugs in zumindest eine von der Axialrichtung abweichende Richtung zu bewegen. Unter einem "Spannmodus" soll hier insbesondere ein Zustand der Spanneinheit verstanden werden, in dem das Bearbeitungswerkzeug mittels des Spannelements und/oder des Spannkopfs gesichert ist. Insbesondere ist das Bearbeitungswerkzeug im Spannmodus mittels der Spanneinheit zu einer Bearbeitung von Werkstücken an einer Werkzeugaufnahme einer tragbaren Werkzeugmaschine entlang der Axialrichtung und/oder entlang der Radialrichtung gesichert. Hierbei wird das Bearbeitungswerkzeug besonders bevorzugt mit einer Spannkraft entlang der Axialrichtung beaufschlagt. Unter einer "Radialrichtung" soll hier insbesondere eine zumindest im Wesentlichen senkrecht zur Axialrichtung verlaufende Richtung verstanden werden. Bevorzugt ist das Bewegungswandlungselement getrennt von dem Spannelement und/oder dem Spannkopf ausgebildet. Der Ausdruck "axiale Überdeckung" soll hier insbesondere eine Überlappung, insbesondere von Teilbereichen, von zumindest zwei Bauteilen entlang der Axialrichtung definieren, insbesondere schneidet eine Gerade entlang der Axialrichtung die zwei Bauteile. Zumindest eine Spannfläche des Spannkopfs überdeckt zumindest einen Teilbereich des Bearbeitungswerkzeugs entlang der Axialrichtung im Spannmodus. Besonders bevorzugt kann die Überdeckung in einem montierten Zustand des Spannkopfs temporär aufgehoben werden, insbesondere zum Wechsel des Bearbeitungswerkzeugs und/oder zu einer Stellungsänderung, insbesondere eine Winkelstellungsänderung, des Bearbeitungswerkzeugs. Es kann vorteilhaft ein werkzeugloses Festspannen und/oder Lösen des Bearbeitungswerkzeugs erreicht werden. Somit kann vorteilhaft auf ein zusätzliches Werkzeug zum Festspannen und/oder zum Lösen verzichtet werden. Ferner kann eine Montage und/oder ein Festspannen des Bearbetiungswerkzeugs an der Werkzeugaufnahme der tragbaren Werkzeugmaschine entkoppelt von einer Entnahme und/oder einer Demontage des Spannelements erfolgen.

Vorteilhafterweise ist das Bewegungswandlungselement als Bolzen ausgebildet. Unter einem "Bolzen" soll hier insbesondere ein Element verstanden werden, das eine Längserstreckung aufweist, die größer ist als eine senkrecht zur Längserstreckung verlaufende Quererstreckung. Bevorzugt ist der Bolzen zylinderförmig ausgebildet. Besonders bevorzugt ist der Bolzen rotationssymmetrisch um zumindest eine Achse ausgebildet. Vorzugsweise ist der Bolzen aus einem Vollmaterial gebildet. Es ist jedoch auch denkbar, dass das Bewegungswandlungselement eine andere, einem Fachmann als sinnvolle erscheinende Ausgestaltung aufweist. Es kann konstruktiv einfach ein Bewegungswandlungselement erreicht werden. Insbesondere bei einer Ausgestaltung des Bolzens als zylinderförmiger Bolzen kann ferner besonders vorteilhaft ein Gleiten zumindest eines Teilbereichs des Spannkopfs am Bewegungswandlungselement bei einer Bewegung des Spannkopfs infolge einer Bewegung des Spannelements erfolgen. Es kann vorteilhaft eine materialschonende erfindungsgemäße Werkzeugspanneinrichtung erreicht werden. Somit kann vorteilhaft eine hohe Lebensdauer der erfindungsgemäßen Werkzeugspanneinrichtung erreicht werden.

Vorzugsweise ist der Spannkopf federnd an dem Spannelement angeordnet. Der Ausdruck "federnd angeordnet" soll hier insbesondere eine gelenkfreie federelastische Anbindung eines Elements an einem weiteren Element definieren, wobei eine Relativbewegung der Elemente entkoppelt von einem Gelenk und infolge einer elastischen Auslenkung der Elemente relativ zueinander möglich ist. Bevorzugt wird die federnde Anordnung durch spezielle Materialeigenschaften und/oder eine spezielle Geometrie des Spannkopfs und des Spannelements realisiert. Insbesondere kann der Spannkopf relativ zum Spannelement mehr als 1 mm, bevorzugt mehr als 2 mm und besonders bevorzugt mehr als 5 mm elastisch ausgelenkt werden. Es kann vorteilhaft konstruktiv einfach eine Bewegungsmöglichkeit des Spannkopfs relativ zum Spannelement erreicht werden.

Zudem wird vorgeschlagen, dass die Bewegungswandlungseinheit zumindest ein Kurvenglied aufweist, in das das Bewegungswandlungselement zumindest teilweise eingreift. Unter einem "Kurvenglied" soll hier insbesondere ein Element eines Kurvengetriebes verstanden werden. Der Begriff "Kurvengetriebe" soll hier insbesondere einen Mechanismus definieren, der infolge einer Bewegung des Kurvenglieds und infolge eines Zusammenwirkens mit dem Bewegungswandlungselement ein Bauteil ansteuert, das infolgedessen eine durch das Zusammenwirken des Kurvenglieds und des Bewegungswandlungselements vorgegebene Bewegung ausführt. Es kann konstruktiv einfach eine Bewegung eines Elements in eine Bewegung eines weiteren Elements umgewandelt werden. Somit kann vorteilhaft eine einfach zu bedienende Werkzeugspannvorrichtung erreicht werden.

Vorzugsweise ist das Kurvenglied am Spannelement angeordnet. Besonders bevorzugt ist das Kurvenglied als Nut ausgebildet. Die Nut wird insbesondere von einer Materialaussparung im Spannelement gebildet. Bevorzugt weist das Kurvenglied im Vergleich zu einem an das Kurvenglied angrenzenden Bereich des Spannelements eine geringere Materialstärke auf, insbesondere eine Materialstärke von 0 mm. Es ist jedoch auch denkbar, dass zumindest zwei Rippen an einer Außenfläche des Spannelements angeordnet sind, in die das Bewegungswandlungselement zumindest teilweise eingreift. Die zumindest zwei. Rippen können sich hierbei insbesondere ausgehend von der Außenfläche zumindest im Wesentlichen senkrecht in eine von dem Spannelement weg gerichtete Richtung erstrecken, so dass die zumindest zwei Rippen eine Führung für das Bewegungswandlungselement bilden können. Andere, einem Fachmann als sinnvoll erscheinende Ausgestaltungen zur Bildung eines Kurvenglieds sind ebenfalls denkbar. Das Bewegungswandlungselement erstreckt sich bevorzugt durch das Kurvenglied hindurch, insbesondere entlang einer zumindest im Wesentlichen senkrecht zur Axialrichtung verlaufenden Richtung. Unter dem Begriff "hindurch erstrecken" soll hier insbesondere verstanden werden, dass das Bewegungswandlungselement eine Haupterstreckung entlang einer zumindest im Wesentlichen senkrecht zur Axialrichtung verlaufenden Radialrichtung aufweist, die größer ist als eine Erstreckung des Kurvenglieds entlang der zumindest im Wesentlichen senkrecht zur Axialrichtung verlaufenden Radialrichtung. Es kann vorteilhaft Bauraum eingespart werden. Somit kann vorteilhaft eine kompakte Werkzeugspannvorrichtung erreicht werden.

Ferner wird vorgeschlagen, dass zumindest der Spannkopf zumindest zwei relativ zueinander bewegliche Teilbereiche aufweist. Bevorzugt sind die zwei Teilbereiche infolge einer elastischen Materialeigenschaft und/oder einer speziellen Geometrie des Spannkopfs relativ zueinander beweglich. Bevorzugt sind die zwei Teilbereiche infolge einer federnden Anbindung des Spannkopfs am Spannelement relativ zueinander beweglich. In einer alternativen Ausgestaltung der erfindungsgemäßen Werkzeugspannvorrichtung weist das Spannelement ebenfalls zwei zueinander bewegliche Teilbereiche auf. Hierbei sind die Teilbereiche des Spannelements relativ zueinander schwenkbar gelagert. Hierbei ist besonders bevorzugt jeweils ein Teilbereich des Spannkopfs einstückig mit einem Teilbereich des Spannelements ausgebildet. Es kann vorteilhaft eine symmetrische Verspannung des Bearbeitungswerkzeugs erreicht werden, insbesondere an einer Werkzeugaufnahme einer tragbaren Werkzeugmaschine.

Die Bewegungswandlungseinheit ist als Spreizeinheit ausgebildet, die dazu vorgesehen ist, zumindest zwei relativ zueinander bewegliche Teilbereiche des Spannkopfs in zumindest einem Betriebsmodus der Spanneinheit relativ zueinander in entgegengesetzt gerichtete Richtungen zu bewegen. Bevorzugt werden die Teilbereiche des Spannkopfs und/oder des Spannelements entlang einer Hauptbewegungskomponente bewegt, die zumindest im Wesentlichen senkrecht zur Axialrichtung verläuft. Es kann konstruktiv einfach eine Bewegung von zwei Teilbereichen des Spannkopfs und/oder des Spannelements erreicht werden.

Vorzugsweise ist der Spannkopf einstückig mit dem Spannelement ausgebildet. Unter "einstückig" soll hier insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Es kann vorteilhaft Montageaufwand eingespart werden.

Unter einer "tragbaren Werkzeugmaschine" soll hier insbesondere eine Werkzeugmaschine, insbesondere eine Handwerkzeugmaschine, verstanden werden, die von einem Bediener transportmaschinenlos transportiert werden kann. Die tragbare Werkzeugmaschine weist insbesondere eine Masse auf, die kleiner ist als 40 kg, bevorzugt kleiner als 10 kg und besonders bevorzugt kleiner als 5 kg. Es kann vorteilhaft ein hoher Bedienkomfort für einen Bediener der Werkzeugmaschine erreicht werden.

Die Werkzeugspannvorrichtung soll hierbei nicht auf die oben beschriebene Anwendung und Ausführungsform beschränkt sein. Insbesondere kann die erfindungsgemäße Werkzeugspannvorrichtung zu einer Erfüllung einer hierin beschriebenen Funktionsweise eine von einer hierin genannten Anzahl von einzelnen Elementen, Bauteilen und Einheiten abweichende Anzahl aufweisen.

### Zeichnung

Weitere Vorteile ergeben sich aus der folgenden Zeichnungsbeschreibung. In der Zeichnung sind Ausführungsbeispiele der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine erfindungsgemäße Werkzeugmaschine mit einer Werkzeugspannvorrichtung in einer schematischen Darstellung,
- Fig. 2: eine Detailansicht der Werkzeugspannvorrichtung in einem Werkzeugwechselmodus einer Spanneinheit der Werkzeugspannvorrichtung in einer schematischen Darstellung,
- Fig. 3: eine Detailansicht der Werkzeugspannvorrichtung in einem Spannmodus der Spanneinheit in einer schematischen Darstellung,
- Fig. 4: eine Detailansicht einer alternativen Werkzeugspannvorrichtung in einem Werkzeugwechselmodus einer Spanneinheit der alternativen Werkzeugspannvorrichtung in einer schematischen Darstellung,
- Fig. 5: eine Detailansicht eines Spannkopfs einer Spanneinheit der alternativen Werkzeugspannvorrichtung in einem Werkzeugwechselmodus der Spanneinheit in einer schematischen Darstellung,
- Fig. 6: eine Detailansicht der alternativen Werkzeugspannvorrichtung aus Figur 4 in einem Spannmodus der Spanneinheit in einer schematischen Darstellung und
- Fig. 7: eine Detailansicht des Spannkopfs der Spanneinheit der alternativen Werkzeugspannvorrichtung in einem Spannmodus der Spanneinheit in einer schematischen Darstellung.

### Beschreibung der Ausführungsbeispiele

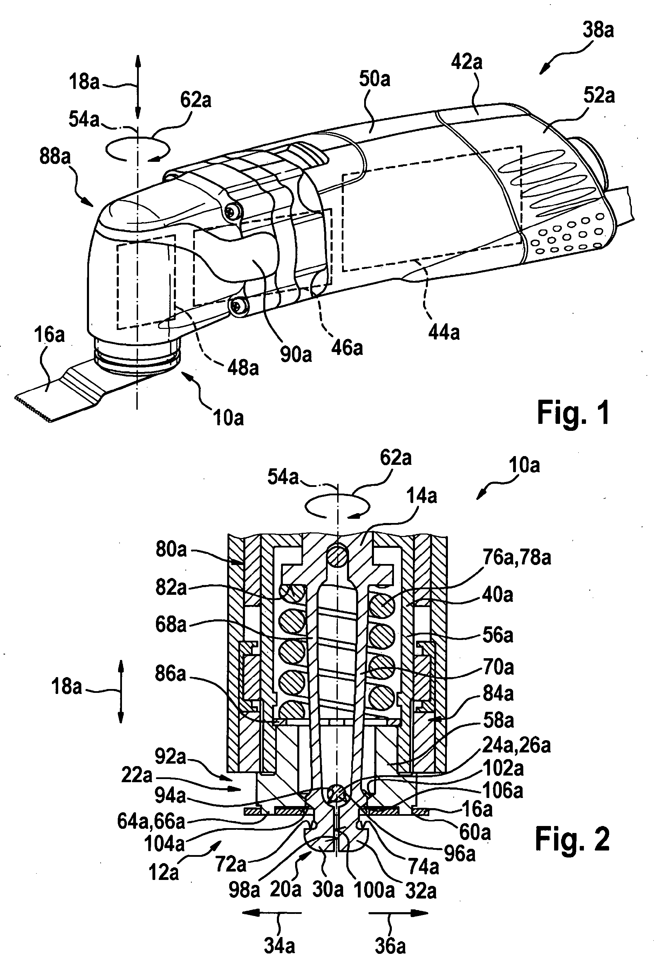

Figur 1 zeigt eine elektrisch betriebene tragbare Werkzeugmaschine 38a mit einer Werkzeugspannvorrichtung 10a. Die tragbare Werkzeugmaschine 38a umfasst ein Werkzeugmaschinengehäuse 42a, das eine Elektromotoreinheit 44a, eine Getriebeeinheit 46a und eine Abtriebseinheit 48a der tragbaren Werkzeugmaschine 38a umschließt. Das Werkzeugmaschinengehäuse 42a umfasst hierbei zwei Gehäusehalbschalen 50a, 52a, die lösbar entlang einer durch eine Axialrichtung 18a verlaufenden Ebene miteinander verbunden sind. Es ist jedoch auch denkbar, dass das Werkzeugmaschinengehäuse 42a zwei oder mehr topfförmige Gehäuseteile aufweist, die lösbar miteinander verbindbar sind. Die Axialrichtung 18a verläuft entlang und/oder parallel zu einer Rotationsachse 54a einer als Spindel 40a ausgebildeten Hohlwelle 56a der Abtriebseinheit 48a (Figur 2). Die Hohlwelle 56a ist dazu vorgesehen, in einem montierten Zustand ein Bearbeitungswerkzeug 16a oszillierend anzutreiben. Ein oszillierender Antrieb des Bearbeitungswerkzeugs 16a erfolgt hierbei auf eine, einem Fachmann bereits bekannte Art und Weise, wie beispielsweise mittels eines exzentrisch auf einer Antriebswelle der Elektromotoreinheit 44a angeordneten Zapfens (hier nicht näher dargestellt) der Getriebeeinheit 46a, der mittels einer Schwinge und einer Schwinghülse (hier nicht näher dargestellt) der Getriebeeinheit 46a die Hohlwelle 56a in einem Betrieb der tragbaren Werkezugmaschine 38a antreibt. Somit ist die als Spindel 40a ausgebildete Hohlwelle 56a oszillierend antreibbar. Das Bearbeitungswerkzeug 16a ist zur spanenden Bearbeitung von Werkstücken an einer Werkzeugaufnahme 58a der Abtriebseinheit 48a befestigbar. Die Werkzeugaufnahme 58a ist mittels einer formschlüssigen und/oder kraftschlüssigen Verbindung drehfest mit der Hohlwelle 56a verbunden. Es ist jedoch auch denkbar, dass die Werkzeugaufnahme 58a einstückig mit der Hohlwelle 56a ausgebildet ist. Es kann eine Schwenkbewegung der Hohlwelle 56a auf die Werkzeugaufnahme 58a übertragen werden.

Figur 2 zeigt eine Detailansicht der Werkzeugspannvorrichtung 10a in einem Werkzeugwechselmodus, in dem ein Bediener das Bearbeitungswerkzeug 16a an der Werkzeugaufnahme 58a montieren kann und/oder eine Stellung des Bearbeitungswerkzeugs 16a entlang einer Umfangsrichtung 62a relativ zur Werkzeugaufnahme 58a ändern kann. Zur drehfesten Befestigung des Bearbeitungswerkzeugs 16a an der Werkzeugaufnahme 58a weist das Bearbeitungswerkzeug 16a Mitnahmeausnehmungen 60a auf, die in einem Kreisring entlang der Umfangsrichtung 62a gleichmäßig verteilt am Bearbeitungswerkzeug 16a angeordnet sind. Insgesamt weist das Bearbeitungswerkzeug 16a 12 Mitnahmeausnehmungen 60a (hier nicht alle dargestellt) auf, die entlang der Umfangsrichtung 62a gleichmäßig verteilt in einem Kreisring angeordnet sind. Es ist jedoch auch denkbar, dass das Bearbeitungswerkzeug 16a eine von 12 abweichende Anzahl an Mitnahmeausnehmungen 60a aufweist. Die Umfangsrichtung 62a verläuft in einer sich senkrecht zur Axialrichtung 18a erstreckenden Ebene. Die Werkzeugaufnahme 58a weist mit den Mitnahmeausnehmungen 60a korrespondierende höckerartige Erhebungen 64a auf, die sich in einem montierten Zustand des Bearbeitungswerkzeugs 16a an der Werkzeugaufnahme 58a entlang der Axialrichtung 18a durch die Mitnahmeausnehmungen 60a hindurch erstrecken. Die höckerartigen Erhebungen 64a sind hierbei als Rastnocken 66a ausgebildet. Insgesamt weist die Werkzeugaufnahme 58a 12 Rastnocken 66a (hier nicht alle dargestellt) auf, die entlang der Umfangsrichtung 62a gleichmäßig verteilt in einem Kreisring angeordnet sind. Es ist jedoch auch denkbar, dass die Werkzeugaufnahme 58a eine von 12 abweichende Anzahl an Rastnocken 66a aufweist.

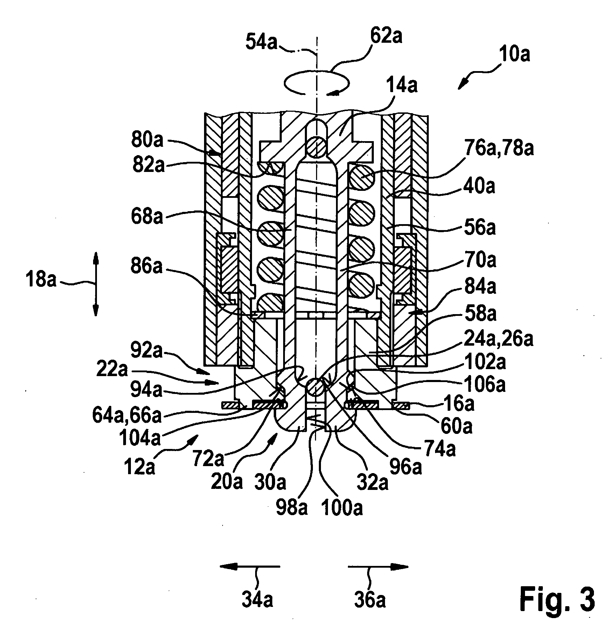

Die Werkzeugspannvorrichtung 10a umfasst eine Spanneinheit 12a. Die Spanneinheit 12a weist ein stiftförmiges Spannelement 14a zu einem Festspannen des Bearbeitungswerkzeugs 16a in Axialrichtung 18a auf (Figur 3). Das Spannelement 14a ist beweglich in der Hohlwelle 56a angeordnet. Hierbei erstreckt sich das Spannelement 14a entlang der Axialrichtung 18a durch die Hohlwelle 56a hindurch. Somit ist das Spannelement 14a in einem montierten Zustand in der Hohlwelle 56a angeordnet. Des Weiteren weist das Spannelement 14a zwei Schenkel 68a, 70a auf, die sich in einem montierten Zustand des Spannelements 14a zumindest im Wesentlichen entlang der Axialrichtung 18a erstrecken. Die Schenkel 68a, 70a sind einstückig mit dem Spannelement 14a ausgebildet. Ferner weisen die Schenkel 68a, 70a eine geringe Materialstärke, entlang einer senkrecht zur Axialrichtung 18a verlaufenden Richtung betrachtet, zur Ermöglichung einer Auslenkung der Schenkel 68a, 70a auf. Somit sind die Schenkel 68a, 70a infolge von Materialeigenschaften und/oder einer geometrischen Form der Schenkel 68a, 70a relativ zueinander beweglich am Spannelement 14a angeordnet. Die Schenkel 68a, 70a sind hierbei federnd am Spannelement 14a angeordnet. Des Weiteren sind die Schenkel 68a, 70a entlang der senkrecht zur Axialrichtung 18a verlaufenden Richtung relativ zueinander beabstandet angeordnet. Die Schenkel 68a, 70a können sich infolge der federnden Anordnung am Spannelement 14a und dem relativen Abstand zueinander entlang der senkrecht zur Axialrichtung 18a verlaufenden Richtung relativ zueinander bewegen.

Ferner weist die Spanneinheit 12a einen an dem Spannelement 14a angeordneten Spannkopf 20a auf. Hierbei ist der Spannkopf 20a einstückig mit dem Spannelement 14a ausgebildet. Der Spannkopf 20a weist zwei relativ zueinander bewegliche Teilbereiche 30a, 32a auf. Jeweils einer der Teilbereiche 30a, 32a ist an einem Schenkel 68a, 70a des Spannelements 14a angeordnet. Hierbei sind die Teilbereiche 30a, 32a des Spannkopfs 20a jeweils einstückig mit einem der Schenkel 68a, 70a des Spannelements 14a ausgebildet. Somit ist der Spannkopf 20a mittels den Schenkeln 68a, 70a federnd an dem Spannelement 14a angeordnet. Die Teilbereiche 30a, 32a weisen zudem jeweils eine Spannfläche 72a, 74a auf, die in einem Spannmodus der Spanneinheit 12a zum Festspannen des Bearbeitungswerkzeugs 16a in Axialrichtung 18a zumindest an einer Teilfläche des Bearbeitungswerkzeugs 16a anliegt. Die Spannflächen 72a, 74a sind im Spannmodus dazu vorgesehen, das Bearbeitungswerkzeug 16a im Spannmodus mit einer Spannkraft entlang der Axialrichtung 18a zu beaufschlagen.

Des Weiteren weist die Spanneinheit 12a ein Federelement 76a auf, das dazu vorgesehen ist, das Spannelement 14a entlang der Axialrichtung 18a mit einer Federkraft zu beaufschlagen. Das Federelement 76a ist hierbei als Druckfeder 78a ausgebildet. Es ist jedoch auch denkbar, dass das Federelement 76a von einem anderen, einem Fachmann als sinnvoll erscheinenden Federelement gebildet wird, wie beispielsweise einer Zugfeder, einer Tellerfeder usw. Ferner ist es ebenfalls denkbar, dass die Spanneinheit 12a mehr als ein Federelement 76a zur Beaufschlagung des Spannelements 14a mit einer Federkraft aufweist. Das Spannelement 14a erstreckt sich in einem montierten Zustand entlang der Axialrichtung 18a durch die Druckfeder 78a hindurch. Somit ist die Druckfeder 78a entlang der Umfangsrichtung 62a zumindest um einen Teilbereich des Spannelements 14a angeordnet. Die Druckfeder 78a stützt sich in einem montierten Zustand mit einem Ende 80a an einer Anlagefläche 82a des Spannelements 14a ab. Die Anlagefläche 82a ist hierbei kreisringförmig ausgebildet. Ferner stützt sich die Druckfeder 78a mit einem weiteren Ende 84a an einem an der Werkzeugaufnahme 58a angeordneten Sicherungsring 86a ab. Der Sicherungsring 86a ist zur Vorspannung der Druckfeder 78a vorgesehen. Hierbei ist der Sicherungsring 86a in einem montierten Zustand in einer Nut in der Hohlwelle 56a angeordnet. Das weitere Ende 84a ist an einer Seite der Druckfeder 78a angeordnet, die einer Seite der Druckfeder 78a gegenüberliegt, an der sich die Druckfeder 78a mit dem Ende 80a an der Anlagefläche 82a des Spannelements 14a abstützt. Die Druckfeder 78a ist somit, entlang der Axialrichtung 18a betrachtet, zwischen der Anlagefläche 82a des Spannelements 14a und der an der Werkzeugaufnahme 58a angeordneten Sicherungsring 86a angeordnet. Der Sicherungsring 86a ist hierbei an einer Seite der Werkzeugaufnahme 58a angeordnet, die, entlang der Axialrichtung 18a betrachtet, einem montierten Bearbeitungswerkzeug 16a abgewandt ist.

Zur Betätigung des Spannelements 14a weist die Spanneinheit 12a eine Bedieneinheit 88a auf (Figur 1). Die Bedieneinheit 88a umfasst einen Bedienhebel 90a, der drehbar um die Rotationsachse 54a der Hohlwelle 56a gelagert ist. Ferner weist die Bedieneinheit 88a einen Mechanismus (hier nicht näher dargestellt) auf, der dazu vorgesehen ist, eine Drehbewegung des Bedienhebels 90a um die Rotationsachse 54a in eine translatorische Bewegung des Spannelements 14a entlang der Axialrichtung 18a umzuwandeln. Der Mechanismus kann hierbei von einem Getriebe, einer Steuerkurve oder anderen, einem Fachmann bereits bekannten Mechanismen zur Umsetzung einer Drehbewegung in eine translatorische Bewegung gebildet werden. Die Bedieneinheit 88a ist im Spannmodus von einer oszillierenden Bewegung des Spannelements14a während eines Betriebs der tragbaren Werkzeugmaschine 38a auf eine dem Fachmann bereits bekannte Art und Weise entkoppelt. Im Werkzeugwechselmodus wird die Bedieneinheit 88a auf eine dem Fachmann bereits bekannte Art und Weise mit dem Spannelement 14a und/oder dem Spannkopf 20a gekoppelt, um eine Spannkraft zu lösen. Der Bediener kann das Bearbeitungswerkzeug 16a nach einem Lösen der Spannkraft des Spannelements 14a und/oder des Spannkopfs 20a von der Werkzeugaufnahme 58a abnehmen.

Die Werkzeugspannvorrichtung 10a weist ferner eine Bewegungswandlungseinheit 22a auf, die dazu vorgesehen ist, den Spannkopf 20a in Abhängigkeit einer Bewegung des Spannelements 14a zumindest im Wesentlichen entlang der Axialrichtung 18a in eine von der Axialrichtung 18a abweichende Richtung zu bewegen. Der Spannkopf 20a wird hierbei mittels der Bewegungswandlungseinheit 22a um eine senkrecht zur Axialrichtung 18a verlaufende Achse geschwenkt. Hierbei wird der Spannkopf 20a zumindest im Wesentlichen entlang einer Hauptbewegungsrichtung bewegt, die zumindest im Wesentlichen senkrecht zur Axialrichtung 18a verläuft. Die Bewegungswandlungseinheit 22a ist als Spreizeinheit 92a ausgebildet, die dazu vorgesehen ist, die zwei relativ zueinander beweglichen Teilbereiche 30a, 32a des Spannkopfs 20a in einem Betriebsmodus der Spanneinheit 12a relativ zueinander in entgegengesetzt gerichtete Richtungen 34a, 36a zu bewegen. Die Bewegungswandlungseinheit 22a weist hierbei ein Bewegungswandlungselement 24a auf, das dazu vorgesehen ist, im Spannmodus der Spanneinheit 12a den Spannkopf 20a zur Erzielung einer axialen Überdeckung des Spannkopfs 20a und des Bearbeitungswerkzeugs 16a in eine von der Axialrichtung 18a abweichende Richtung zu bewegen. Das Bewegungswandlungselement 24a ist als ein Bolzen 26a ausgebildet. Es ist jedoch auch denkbar, dass das Bewegungswandlungselement 24a als Stift und/oder als geschlitzter Stift ausgebildet ist. Andere, einem Fachmann als sinnvoll erscheinende Ausgestaltungen des Bewegungswandlungselements 24a sind ebenfalls denkbar.

Zur Montage des Bearbeitungswerkzeugs 16a an der Werkzeugaufnahme 58a wird der Bedienhebel 90a, ausgehend von einer am Werkzeugmaschinengehäuse 42a anliegenden Position des Bedienhebels 90a, von dem Bediener in eine von dem Werkzeugmaschinengehäuse 42a weg gerichtete Richtung bewegt und somit um die Rotationsachse 54a gedreht. Hierdurch wird das Spannelement 14a entlang der Axialrichtung 18a in Richtung der Werkzeugaufnahme 58a bewegt. Die Spanneinheit 12a befindet sich im Werkzeugwechselmodus. Der Bediener kann unter Beibehaltung der Verdrehung des Bedienhebels 90a um die Rotationsachse 54a das Bearbeitungswerkzeug 16a an der Werkzeugaufnahme 58a anbringen. Es ist jedoch auch denkbar, dass die Bedieneinheit 88a eine Rastvorrichtung (hier nicht näher dargestellt) aufweist, die dazu vorgesehen ist, den Bedienhebel 90a in einer Position, wie beispielsweise in einer dem Werkzeugwechselmodus entsprechenden Position des Bedienhebels 90a, zu arretieren. Das Bearbeitungswerkzeug 16a wird von dem Bediener mit einer zentralen Einführöffnung über den Spannkopf 20a geführt, bis das Bearbeitungswerkzeug 16a an der Werkzeugaufnahme 58a anliegt und die Rastnocken 66a der Werkzeugaufnahme 58a in den Mitnahmeausnehmungen 60a des Bearbeitungswerkzeugs 16a angeordnet sind. Das Bearbeitungswerkzeug 16a wird hierdurch im Spannmodus der Spanneinheit 12a gegen ein Verdrehen entlang der Umfangsrichtung 62a gesichert. Nachdem eine Krafteinwirkung des Bedieners auf den Bedienhebel 90a aufgehoben wird, wird der Bedienhebel 90a infolge einer Federkraft der Druckfeder 78a, die über den Mechanismus zur Umwandlung der Drehbewegung des Bedienhebels 90a in eine translatorische Bewegung des Spannelements 14a auf den Bedienhebel 90a einwirkt, in eine Ausgangsposition in Richtung des Werkzeugmaschinengehäuses 42a bewegt. Die Spanneinheit 12a befindet sich somit im Spannmodus.

Bei einer Bewegung des Spannelements 14a entlang der Axialrichtung 18a in eine von der Werkzeugaufnahme 58a weg gerichtete Richtung werden die zwei Teilbereiche 30a, 32a des Spannkopfs 20a auf das als Bolzen 26a ausgebildete Bewegungswandlungselement 24a der als Spreizeinheit 92a ausgebildeten Bewegungswandlungseinheit 22a zu bewegt. Sobald die Teilbereiche 30a, 32a während der Bewegung entlang der Axialrichtung 18a zur Anlage mit dem Bolzen 26a kommen, gleitet jeweils eine Gleitfläche 94a, 96a der Teilbereiche 30a, 32a entlang einer Außenfläche des Bolzens 26a. Die Gleitflächen 94a, 96a weisen einen kreisbogenförmigen Verlauf auf. Ferner sind die Gleitflächen 94a, 96a an einer der Druckfeder 78a zugewandten Seite der Teilbereiche 30a, 32a angeordnet. Die Teilbereiche 30a, 32a werden infolge der Bewegung entlang der Axialrichtung 18a und des Gleitens der Gleitflächen 94a, 96a entlang der Außenfläche des Bolzens 26a in die zwei zueinander entgegengesetzt ausgerichtete Richtungen 34a, 36a bewegt, bis der Bolzen 26a infolge der Bewegung des Spannelements 14a zwischen den Teilbereichen 30a, 32a an jeweils einer Spreizfläche 98a, 100a der Teilbereiche 30a, 32a anliegt. Der Bolzen 26a ist, entlang einer senkrecht zur Axialrichtung 18a verlaufenden Richtung betrachtet, im Spannmodus zwischen den Spreizflächen 98a, 100a der Teilbereiche 30a, 32a angeordnet. Der Spannkopf 20a wird somit mittels des Bewegungswandlungselements 24a gespreizt. Die Spannflächen 72a, 74a der Teilbereiche 30a, 32a liegen nach einem beendeten Spannvorgang an Teilflächen des Bearbeitungswerkzeugs 16a an und beaufschlagen das Bearbeitungswerkzeug 16a mit einer Spannkraft entlang der Axialrichtung 18a in Richtung der Werkzeugaufnahme 58a.

Bei einem Lösevorgang der Spanneinheit 12a wird die Spanneinheit 12a von dem Bediener aus dem Spannmodus in den Werkzeugwechselmodus überführt. Hierbei wird der Bedienhebel 90a von dem Bediener aus der Ausgangsposition in die vom Werkzeugmaschinengehäuse 42a weg gerichtete Richtung bewegt und um die Rotationsachse 54a gedreht. Hierbei wird das Spannelement 14a entlang der Axialrichtung 18a in Richtung der Werkzeugaufnahme 58a bewegt. Infolge . der Bewegung des Spannelements 14a entlang der Axialrichtung 18a in Richtung der Werkzeugaufnahme 58a gleiten die Spreizflächen 98a, 100a der Teilbereiche 30a, 32a an der Außenfläche des Bolzens 26a, bis der Bolzen 26a außer Eingriff der Spreizflächen 98a, 100a gelangt. Die Teilbereiche 30a, 32a werden infolge der federnden Anordnung am Spannelement 14a aufeinander zu bewegt. Zur Unterstützung der Bewegung der Teilbereiche 30a, 32a weist die Bewegungswandlungseinheit 22a eine Schrägfläche 102a auf, die korrespondierend mit jeweils einer Schrägfläche 104a, 106a der Teilbereiche 30a, 32a ausgebildet ist. Sobald der Bolzen 26a außer Eingriff der Spreizflächen 98a, 100a ist, liegen die Schrägfläche 102a der Bewegungswandlungseinheit 22a und die Schrägflächen 104a, 106a der Teilbereiche 30a, 32a des Spannkopfs 20a aneinander an. Mittels eines Zusammenwirkens der Schrägfläche 102a der Bewegungswandlungseinheit 22a und den Schrägflächen 104a, 106a der Teilbereiche 30a, 32a und infolge der Bewegung des Spannelements 14a entlang der Axialrichtung 18a in Richtung der Werkzeugaufnahme 58a werden die Teilbereiche 30a, 32a weiter aufeinander zu bewegt. Hierbei wird eine axiale Überdeckung entlang der Axialrichtung 18a der Spannflächen 72a, 74a der Teilbereiche 30a, 32a und des Bearbeitungswerkzeugs 16a aufgehoben. Somit kann das Bearbeitungswerkzeug 16a von der Werkzeugaufnahme 58a entnommen werden.

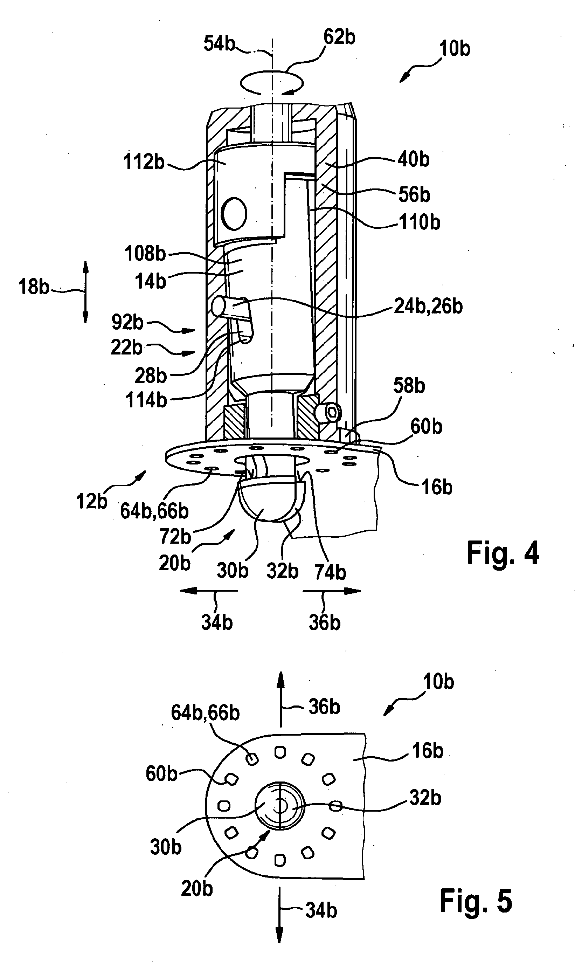

In Figuren 4 bis 7 ist ein alternatives Ausführungsbeispiel dargestellt. Im Wesentlichen gleich bleibende Bauteile, Merkmale und Funktionen sind grundsätzlich mit den gleichen Bezugszeichen beziffert. Zur Unterscheidung der Ausführungsbeispiele sind den Bezugszeichen der Ausführungsbeispiele die Buchstaben a und b hinzugefügt. Die nachfolgende Beschreibung beschränkt sich im Wesentlichen auf die Unterschiede zu dem ersten Ausführungsbeispiel in den Figuren 1 bis 3, wobei bezüglich gleich bleibender Bauteile, Merkmale und Funktionen auf die Beschreibung des ersten Ausführungsbeispiels in den Figuren 1 bis 3 verwiesen werden kann.

Figur 4 zeigt eine Detailansicht einer Werkzeugspannvorrichtung 10b in einem Werkzeugwechselmodus einer Spanneinheit 12b der Werkzeugspannvorrichtung 10b. Die Werkzeugspannvorrichtung 10b ist in einer tragbaren Werkzeugmaschine (hier nicht näher dargestellt) angeordnet, die einen zur tragbaren Werkzeugmaschine 42a aus der Figur 1 analogen Aufbau aufweist. Die Spanneinheit 12b umfasst ein stiftförmiges Spannelement 14b zu einem Festspannen eines Bearbeitungswerkzeugs 16b in einer Axialrichtung 18b sowie einen an dem Spannelement 14b angeordneten Spannkopf 20b. Des Weiteren weist die Werkzeugspannvorrichtung 10b eine Bewegungswandlungseinheit 22b auf, die dazu vorgesehen ist, den Spannkopf 20b in Abhängigkeit einer Bewegung des Spannelements 14b zumindest im Wesentlichen entlang der Axialrichtung 18b in zumindest eine von der Axialrichtung 18b abweichende Richtung zu bewegen.

Das Spannelement 14b umfasst zwei relativ zueinander bewegliche Teilbereiche 108b, 110b. Die Teilbereiche 108b, 110b des Spannelements 14b sind schwenkbar um eine senkrecht zur Axialrichtung 18b verlaufende Achse in einer als Hohlwelle 56b ausgebildeten Spindel 40b der tragbaren Werkzeugmaschine angeordnet. Zudem sind die Teilbereiche 108b, 110b beweglich entlang der Axialrichtung 18b in der Hohlwelle 56b gelagert. Das Spannelement 14b ist beweglich an einem Übertragungselement 112b der Spanneinheit 12b angeordnet. Das Übertragungselement 112b ist dazu vorgesehen, eine Bewegung eines Bedienelements einer Bedieneinheit (hier nicht näher dargestellt) der Spanneinheit 12b auf das Spannelement 14b zu übertragen.

Der Spannkopf 20b weist ebenfalls zwei relativ zueinander bewegliche Teilbereiche 30b, 32b auf. Die Teilbereiche 30b, 32b des Spannkopfs 20b sind jeweils einstückig mit einem der Teilbereiche 108b, 110b des Spannelements 14b ausgebildet. Ferner sind die Teilbereiche 30b, 32b des Spannkopfs 20b jeweils exzentrisch an dem jeweiligen Teilbereich 108b, 110b des Spannelements 14b angeordnet. Hierbei weisen die Teilbereiche 30b, 32b des Spannkopfs 20b eine Exzentrizität relativ zu einer zumindest in einem Betriebszustand zumindest im Wesentlichen parallel zur Axialrichtung 18b verlaufenden Längsachse des jeweiligen Teilbereichs 108b, 110b des Spannelements 14b auf.

Des Weiteren umfasst die Bewegungswandlungseinheit 22b ein Bewegungswandlungselement 24b, das dazu vorgesehen ist, zumindest in einem Spannmodus der Spanneinheit 12b den Spannkopf 20b zur Erzielung einer axialen Überdeckung des Spannkopfs 20b und des Bearbeitungswerkzeugs 16b in zumindest eine von der Axialrichtung 18b abweichende Richtung zu bewegen. Das Bewegungswandlungselement 24b ist als Bolzen 26b ausgebildet. Ferner umfasst die Bewegungswandlungseinheit 22b zumindest ein Kurvenglied 28b, in das das Bewegungswandlungselement 24b teilweise eingreift. Somit wird die Bewegungswandlungseinheit 22b von einem Kurvengetriebe gebildet. Das Kurvengetriebe ist hierbei als Spreizeinheit 92b ausgebildet, die dazu vorgesehen ist, die zwei relativ zueinander beweglichen Teilbereiche 30b, 32b des Spannkopfs 20b in zumindest einem Betriebsmodus der Spanneinheit 12a relativ zueinander in entgegengesetzt gerichtete Richtungen 34b, 36b zu bewegen.

Das Kurvenglied 28b ist am Spannelement 14b angeordnet. Die Teilbereiche 108b, 110b des Spannelements 14b umfassen jeweils ein Kurvenglied 28b (lediglich ein Kurvenglied in den Figuren dargestellt). Die Kurvenglieder 28b sind als kulissenförmige Nuten 114b (lediglich eine Nut in den Figuren dargestellt) in den Teilbereichen 108b, 110b des Spannelements 14b ausgebildet. Es ist jedoch auch denkbar, dass die Kurvenglieder 28b auf eine andere, einem Fachmann als sinnvoll erscheinende Art und Weise ausgestaltet sind. Die Nuten 114b sind dazu vorgesehen, mittels eines Zusammenwirkens mit dem Bewegungswandlungselement 24b eine Bewegung des jeweiligen Teilbereichs 108b, 110b in Abhängigkeit einer Bewegung des Spannelements 14b entlang der Axialrichtung 18b vorzugeben. Das Bewegungswandlungselement 24b erstreckt sich in einem montierten Zustand entlang einer senkrecht zur Axialrichtung 18b verlaufenden Richtung durch die Nuten 114b der Teilbereiche 108b, 110b hindurch. Das Bewegungswandlungselement 24b ist ortsfest mit der Hohlwelle 56b verbunden.

Bei einer Montage des Bearbeitungswerkzeugs 16b befindet sich die Spanneinheit 12b in einem Werkzeugwechselmodus. Hierbei sind die Teilbereiche 108b, 110b des Spannelements 14b infolge der Bewegungswandlungseinheit 22b relativ zueinander geschwenkt. Somit sind Längsachsen der Teilbereiche 108b, 110b relativ zur Axialrichtung 18b abgewinkelt. Die Teilbereiche 30b, 32b des Spannkopfs 20b sind infolge der exzentrischen Anordnung an den Teilbereichen 108b, 110b des Spannelements 14b im Bereich von Spannflächen 72b, 74b der Teilbereiche 30b, 32b konzentrisch zur Axialrichtung 18b angeordnet (Figur 5). Ein Bediener kann somit das Bearbeitungswerkzeug 16b an einer Werkzeugaufnahme 58b der tragbaren Werkzeugmaschine anbringen. Hierbei wird das Bearbeitungswerkzeug 16b mit einer zentralen Einführöffnung über den Spannkopf 20b geführt, bis das Bearbeitungswerkzeug 16b an der Werkzeugaufnahme 58b anliegt und Erhebungen 64b der Werkzeugaufnahme 58b in Mitnahmeausnehmungen 60b des Bearbeitungswerkzeugs 16b angeordnet sind.

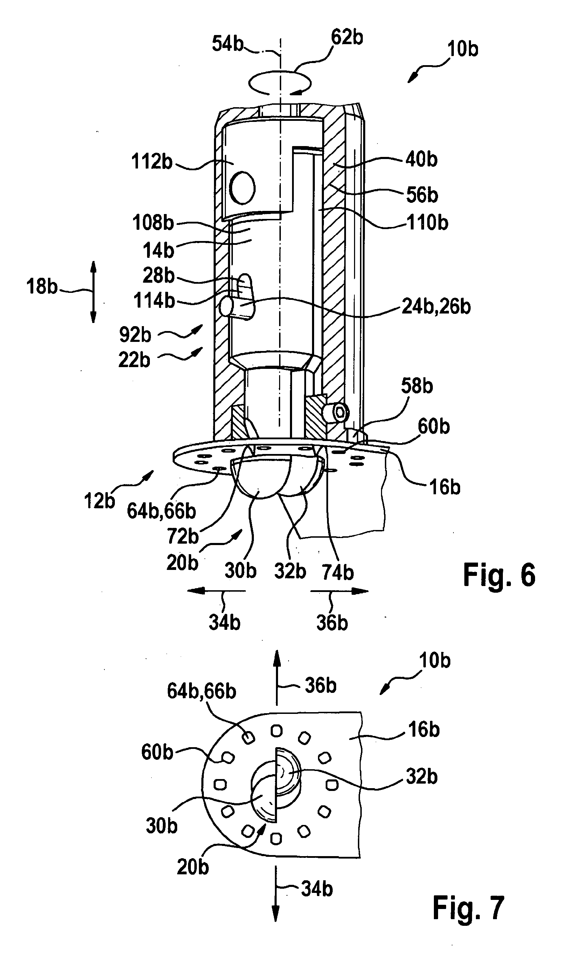

Figur 6 zeigt die Spanneinheit 12b in einem Spannmodus. Bei einer Überführung der Spanneinheit 12b ausgehend von dem Werkzeugwechselmodus in den Spannmodus werden die Teilbereiche 108b, 110b des Spannelements 14b während einer Bewegung des Spannelements 14b entlang der Axialrichtung 18b infolge eines Zusammenwirkens des Bewegungswandlungselements 24b und der Kurvenglieder 28b relativ zueinander geschwenkt. Die Teilbereiche 108b, 110b werden bei einer Bewegung des Spannelements 14b in eine von der Werkzeugaufnahme 58b weg gerichtete Richtung infolge einer Geometrie der Kurvenglieder 28b aufeinander zu geschwenkt. Die Längsachsen der Teilbereiche 108b, 110b sind im Spannmodus parallel zur Axialrichtung 18b ausgerichtet. Somit überdecken sich die Teilbereiche 108b, 110b zumindest im Wesentlichen vollständig entlang einer senkrecht zur Axialrichtung 18b verlaufenden Richtung. Hierbei fluchten jeweils die Außenkanten der Teilbereiche 108b, 110b entlang der senkrecht zur Axialrichtung 18b verlaufenden Richtung miteinander.

Bei einer Schwenkbewegung der Teilbereiche 108b, 110b des Spannelements 14b werden die Teilbereiche 30b, 32b des Spannkopfs 20b relativ zueinander in entgegengesetzt gerichtete Richtungen 34b, 36b bewegt. Die Teilbereiche 30b, 32b des Spannkopfs 20b werden im Wesentlichen entlang einer Hauptbewegungskomponente bewegt, die senkrecht zur Axialrichtung 18b ausgerichtet ist. Infolge der Bewegung der Teilbereiche 30b, 32b des Spannkopfs 20b werden die Spannflächen 72b, 74b der Teilbereiche 30b, 32b an das Bearbeitungswerkzeug 16b angelegt. Somit wird im Spannmodus eine Spannkraft entlang der Axialrichtung 18b auf das Bearbeitungswerkzeug 16b übertragen. Die Teilbereiche 30b, 32b des Spannkopfs 20b sind infolge der exzentrischen Anordnung an den Teilbereichen 108b, 110b im Spannmodus relativ zueinander versetzt angeordnet (Figur 7).

## Patentansprüche

1. Tragbare Werkzeugmaschine, insbesondere tragbare Werkzeugmaschine mit einer oszillierend antreibbaren Spindel, mit zumindest einer Werkzeugspannvorrichtung, insbesondere einer Oszillationswerkzeugspannvorrichtung, mit zumindest einer Spanneinheit (12a; 12b), die zumindest ein stiftförmiges Spannelement (14a; 14b) zu einem Festspannen eines Bearbeitungswerkzeugs (16a; 16b) in einer Axialrichtung (18a; 18b) sowie zumindest einen an dem Spannelement (14a; 14b) angeordneten Spannkopf (20a; 20b) aufweist, der zumindest zwei relativ zueinander bewegliche Teilbereiche (30a, 32a; 30b, 32b) aufweist, und mit einer Bewegungswandlungseinheit (22a; 22b), die dazu vorgesehen ist, die zumindest zwei relativ zueinander beweglichen Teilbereiche (30a, 32a; 30b, 32b) des Spannkopfs (20a; 20b) in Abhängigkeit einer Bewegung des Spannelements (14a; 14b) zumindest im Wesentlichen entlang der Axialrichtung (18a; 18b) in zumindest eine von der Axialrichtung (18a; 18b) abweichende Richtung zu bewegen, wobei die Bewegungswandlungseinheit (22a; 22b) als Spreizeinheit (92a; 92b) ausgebildet ist, die dazu vorgesehen ist, die zumindest zwei relativ zueinander beweglichen Teilbereiche (30a, 32a; 30b, 32b) des Spannkopfs (20a; 20b) in zumindest einem Betriebsmodus der Spanneinheit (12a; 12b) relativ zueinander in entgegengesetzt gerichtete Richtungen (34a, 36a; 34b, 36b) zu bewegen, **dadurch gekennzeichnet, dass** die Teilbereiche (30a, 32a; 30b, 32b) jeweils eine Spannfläche (72a, 74a) aufweisen, die in einem Spannmodus der Spanneinheit (12a; 12b) zum Festspannen des Bearbeitungswerkzeugs (16a; 16b) in Axialrichtung derart gespreizt sind, dass sie zumindest an einer Teilfläche des Bearbeitungswerkzeugs (16a; 16b) anliegen und eine Spannkraft entlang der Axialrichtung (18a; 18b) auf das Bearbeitungswerkzeug (16a; 16b) wirkt.

2. Tragbare Werkzeugmaschine nach Anspruch 1,
**dadurch gekennzeichnet, dass** die Bewegungswandlungseinheit (22a; 22b) zumindest ein Bewegungswandlungselement (24a;24b) aufweist, das dazu vorgesehen ist, zumindest in einem Spannmodus der Spanneinheit (12a; 12b) den Spannkopf (20a; 20b) zur Erzielung einer axialen Überdeckung des Spannkopfs (20a; 20b) und des Bearbeitungswerkzeugs (16a; 16b) in zumindest eine von der Axialrichtung (18a; 18b) abweichende Richtung zu bewegen.

3. Tragbare Werkzeugmaschine nach Anspruch 2
**dadurch gekennzeichnet, dass** das Bewegungswandlungselement (24a; 24b) als Bolzen (26a; 26b) ausgebildet ist.

4. Tragbare Werkzeugmaschine nach einem der vorhergehenden Ansprüche,
**dadurch gekennzeichnet, dass** der Spannkopf (20a) federnd an dem Spannelement (14a) angeordnet ist.

5. Tragbare Werkzeugmaschine zumindest nach Anspruch 2,
**dadurch gekennzeichnet, dass** die Bewegungswandlungseinheit (22b) zumindest ein Kurvenglied (28b) aufweist, in das das Bewegungswandlungselement (24b) zumindest teilweise eingreift.

6. Tragbare Werkzeugmaschine nach Anspruch 5,
**dadurch gekennzeichnet, dass** das Kurvenglied (28b) am Spannelement (14b) angeordnet ist.

7. Tragbare Werkzeugmaschine nach einem der vorhergehenden Ansprüche,
**dadurch gekennzeichnet, dass** der Spannkopf (20a; 20b) einstückig mit dem Spannelement (14a; 14b) ausgebildet ist.

## Claims

1. Portable power tool, in particular a portable power tool having a spindle that can be driven in an oscillatory manner, having at least one tool clamping device, in particular an oscillating-tool clamping device, comprising at least one clamping unit (12a; 12b) that has at least one pin-shaped clamping element (14a; 14b) for chucking a working tool (16a; 16b) in an axial direction (18a; 18b), and has at least one clamping head (20a; 20b) disposed on the clamping element (14a; 14b), which clamping head (20a; 20b) has at least two partial regions (30a, 32a; 30b, 32b) that are movable relative to each other, and comprising a motion conversion unit (22a; 22b), which is provided to move the at least two partial regions (30a, 32a; 30b, 32b) of the clamping head (20a; 20b), which are movable relative to each other, in dependence on a motion of the clamping element (14a; 14b) at least substantially along the axial direction (18a; 18b), in at least one direction differing from the axial direction (18a; 18b), wherein the motion conversion unit (22a; 22b) is realized as an expansion unit (92a; 92b), which is provided to move the at least two partial regions (30a, 32a; 30b, 32b) of the clamping head (20a; 20b), which are movable relative to each other, in oppositely oriented directions (34a, 36a; 34b, 36b) relative to each other, in at least one operating mode of the clamping unit (12a; 12b),
**characterized in that** the partial regions (30a, 32a; 30b, 32b) each have a clamping face (72a, 74a), which, when the clamping unit (12a; 12b) is in a clamping mode, for the purpose of chucking the working tool (16a; 16b) in the axial direction, are expanded such that they bear against at least a partial face of the working tool (16a; 16b), and a clamping force acts on the working tool (16a; 16b) along the axial direction (18a; 18b).

2. Portable power tool according to Claim 1,
**characterized in that** the motion conversion unit (22a; 22b) has at least one motion conversion element (24a; 24b), which is provided to move the clamping head (20a; 20b) in at least one direction differing from the axial direction (18a; 18b), for the purpose of achieving an axial overlap of the clamping head (20a; 20b) and the working tool (16a; 16b), at least when the clamping unit (12a; 12b) is in a clamping mode.

3. Portable power tool according to Claim 2,
**characterized in that** the motion conversion element (24a; 24b) is realized as stud (26a; 26b).

4. Portable power tool according to any one of the preceding claims,
**characterized in that** the clamping head (20a) is disposed in a resilient manner on the clamping element (14a).

5. Portable power tool according to Claim 2,
**characterized in that** the motion conversion unit (22b) has at least one cam member (28b), in which the motion conversion element (24b) engages, at least partially.

6. Portable power tool according to Claim 5,
**characterized in that** the cam member (28b) is disposed on the clamping element (14b).

7. Portable power tool according to any one of the preceding claims,
**characterized in that** the clamping head (20a; 20b) is realized so as to be integral with the clamping element (14a; 14b).

## Revendications

1. Machine-outil portative, en particulier machine-outil portative comprenant une broche pouvant être entraînée en oscillations, comprenant au moins un dispositif de serrage d'outil, en particulier un dispositif de serrage d'outil oscillant, comprenant au moins une unité de serrage (12a ; 12b), qui présente au moins un élément de serrage en forme de broche (14a ; 14b) destiné à insérer fixement un outil d'usinage (16a ; 16b) dans une direction axiale (18a ; 18b) ainsi qu'au moins une tête de serrage (20a ; 20b) disposée sur l'élément de serrage (14a ; 14b), qui présente au moins deux régions partielles (30a, 32a ; 30b, 32b) déplaçables les unes par rapport aux autres, et comprenant une unité de conversion de mouvement (22a ; 22b), qui est prévue pour déplacer au moins deux régions partielles (30a, 32a ; 30b, 32b) de la tête de serrage (20a ; 20b) en fonction d'un mouvement de l'élément de serrage (14a ; 14b) au moins sensiblement le long de la direction axiale (18a ; 18b) dans une direction s'écartant de la direction axiale (18a ; 18b), l'unité de conversion de mouvement (22a ; 22b) étant réalisée sous forme d'unité d'écartement (92a ; 92b) qui est prévue pour déplacer au moins deux régions partielles (30a, 32a ; 30b, 32b) déplaçables les unes par rapport aux autres de la tête de serrage (20a ; 20b) dans au moins un mode de fonctionnement de l'unité de serrage (12a ; 12b) les unes par rapport aux autres dans des directions opposées (34a, 36a ; 34b, 36b), **caractérisée en ce que** les régions partielles (30a, 32a ; 30b, 32b) présentent à chaque fois une surface de serrage (72a, 74a), lesquelles surfaces de serrage, dans un mode de serrage de l'unité de serrage (12a ; 12b) pour serrer fixement l'outil d'usinage (16a ; 16b) dans la direction axiale, sont écartées de telle sorte qu'elles s'appliquent au moins contre une surface partielle de l'outil d'usinage (16a ; 16b) et qu'une force de serrage agisse le long de la direction axiale (18a ; 18b) sur l'outil d'usinage (16a ; 16b).

2. Machine-outil portative selon la revendication 1, **caractérisée en ce que** l'unité de conversion de mouvement (22a ; 22b) présente au moins un élément de conversion de mouvement (24a ; 24b) qui est prévu pour déplacer, au moins dans un mode de serrage de l'unité de serrage (12a ; 12b), la tête de serrage (20a ; 20b) afin d'obtenir un recouvrement axial de la tête de serrage (20a, 20b) et de l'outil d'usinage (16a ; 16b) dans au moins une direction s'écartant la direction axiale (18a ; 18b).

3. Machine-outil portative selon la revendication 2, **caractérisée en ce que** l'élément de conversion de mouvement (24a ; 24b) est réalisé sous forme de boulons (26a ; 26b).

4. Machine-outil portative selon l'une quelconque des revendications précédentes, **caractérisée en ce que** la tête de serrage (20a) est disposée de manière élastique sur l'élément de serrage (14a).

5. Machine-outil portative selon au moins la revendication 2,
**caractérisée en ce que** l'unité de conversion de mouvement (22b) présente au moins un organe de came (28b) dans lequel s'engage au moins en partie l'élément de conversion de mouvement (24b).

6. Machine-outil portative selon la revendication 5, **caractérisée en ce que** l'organe de came (28b) est disposé sur l'élément de serrage (14b).

7. Machine-outil portative selon l'une quelconque des revendications précédentes, **caractérisée en ce que** la tête de serrage (20a ; 20b) est réalisée d'une seule pièce avec l'élément de serrage (14a ; 14b).
